# EUROPEAN PATENT APPLICATION

(11) **EP 3 333 585 A2**
(43) Date of publication of application: **13.06.2018**
(21) Application number: 17206576.5
(22) Date of filing: 12.12.2017
(51) Int. Cl.: G01R 33/561

(54) **A 3D MAGNETIC RESONANCE IMAGING SEQUENCE WITH MAGNETIZATION PREPARATION AND CONTROLLED ALIASING**

(30) Priority: 12.12.2016 US 201662432762 P
(71) Applicant: Siemens Healthcare GmbH, 91052 Erlangen (DE); The General Hospital Corporation, Boston, MA 02114 (US)
(72) Inventor: Beck, Thomas, 91052 Erlangen (DE); Bilgic, Berkin, Boston, MA 02215 (US); Polak, Daniel, 63825 Blankenbach (DE); Setsompop, Kawin, Charlestown, 02129 (US)
(74) Representative: Maier, Daniel Oliver

(57) **Abstract**

A method comprising controlling a radio-frequency transmitter of a magnetic resonance imaging device to apply an inversion pulse to a sample magnetization. In a multi-shot readout phase, the method further comprises controlling a gradient system of the magnetic resonance imaging device to apply a steady-state gradient echo readout sequence comprising at least one first phase-encoding gradient along a first direction, at least one second phase-encoding gradient along a second direction, and a sequence of readout gradients along a readout direction. The method further comprises, in the multi-shot readout phase: controlling the gradient system to apply first AC gradients along the first direction and at least partly contemporaneously with readout gradients of the sequence of readout gradients and controlling the gradient system to apply second AC gradients along the second direction and at least partly contemporaneously with the readout gradients of the sequence of readout gradients.

## Description

### TECHNICAL FIELD

Various examples of the invention generally relate to magnetic resonance imaging. Various examples of the invention specifically relate to three-dimensional magnetic resonance imaging using inner-loop acceleration.

### BACKGROUND

Magnetic resonance imaging (MRI) provides detailed images of the human body. Numerous MRI sequences defined by different use of radio frequency (RF) pulses and magnetic gradient fields (gradients) used for spatial encoding are known. One example is the 3-D Magnetization Prepared Rapid Gradient Echo (3-D MP RAGE) sequence. See Mugler, John P., and James R. Brookeman. "Three-dimensional magnetization-prepared rapid gradient-echo imaging (3-D MP RAGE" Magnetic Resonance in Medicine 15.1 (1990): 152-157, the disclosure of which is incorporated herein in its entirety by reference. For example, 3-D MP Rage allows to obtain MRI images of the brain with detailed contrast between gray matter, white matter, and cerebrospinal fluid.

Conventional implementations of 3-D MP RAGE suffer from significant acquisition times. To mitigate this, techniques of image acceleration are known from Brenner, D., et al. "Two-dimensional accelerated MP-RAGE imaging with flexible linear reordering." Magnetic Resonance Materials in Physics, Biology and Medicine 27.5 (2014): 455-462. These techniques may provide a decreased signal-to-noise ratio (SNR) such that the overall image quality may suffer.

### SUMMARY

Therefore, a need exists for advanced techniques of 3-D MRI. In particular, a need exists for 3-D MRI techniques which overcome or mitigate at least some of the above-identified restrictions and drawbacks.

In an example, a method includes controlling a RF transmitter of a MRI device to apply an inversion pulse to a sample magnetization in a preparation phase. The method further includes controlling a gradient system of the MRI device to apply a steady-state gradient echo readout sequence in a multi-shot readout phase. The steady-state gradient echo readout sequence includes at least one first phase-encoding gradient along the first direction, at least one second phase-encoding gradient along a second direction, and a sequence of readout gradients along the readout direction. The method further includes controlling the gradient system to apply, in the multi-shot readout phase, first time-varying (AC) gradients along the first direction and at least partly contemporaneously with readout gradients of the sequence of readout gradients. The method further includes controlling the gradient system to apply, in the multi-shot readout phase, second AC gradients along the second direction and at least partly contemporaneously with the readout gradients of the sequence of readout gradients. The method further includes controlling an RF receiver of the MRI device to acquire MRI data for the sample magnetization at multiple K-space positions along a K-space trajectory and in the multi-shot readout phase. The first direction may be different from the second direction.

In an example, a computer program product including program code is provided. The program code may be executed by at least one processor. Executing the program code can cause the at least one processor to perform a method. The method includes controlling a RF transmitter of a MRI device to apply an inversion pulse to a sample magnetization in a preparation phase. The method further includes controlling a gradient system of the MRI device to apply a steady-state gradient echo readout sequence in a multi-shot readout phase. The steady-state gradient echo readout sequence includes at least one first phase-encoding gradient along the first direction, at least one second phase-encoding gradient along a second direction , and a sequence of readout gradients along the readout direction. The method further includes controlling the gradient system to apply, in the multi-shot readout phase, first AC gradients along the first direction and at least partly contemporaneously with readout gradients of the sequence of readout gradients. The method further includes controlling the gradient system to apply, in the multi-shot readout phase, second AC gradients along the second direction and at least partly contemporaneously with the readout gradients of the sequence of readout gradients. The method further includes controlling an RF receiver of the MRI device to acquire MRI data for the sample magnetization at multiple K-space positions along a K-space trajectory and in the multi-shot readout phase.

In an example, a computer program including program code is provided. The program code may be executed by at least one processor. Executing the program code can cause the at least one processor to perform a method. The method includes controlling a RF transmitter of a MRI device to apply an inversion pulse to a sample magnetization in a preparation phase. The method further includes controlling a gradient system of the MRI device to apply a steady-state gradient echo readout sequence in a multi-shot readout phase. The steady-state gradient echo readout sequence includes at least one first phase-encoding gradient along the first direction, at least one second phase-encoding gradient along a second direction , and a sequence of readout gradients along the readout direction. The method further includes controlling the gradient system to apply, in the multi-shot readout phase, first AC gradients along the first direction and at least partly contemporaneously with readout gradients of the sequence of readout gradients. The method further includes controlling the gradient system to apply, in the multi-shot readout phase, second AC gradients along the second direction and at least partly contemporaneously with the readout gradients of the sequence of readout gradients. The method further includes controlling an RF receiver of the MRI device to acquire MRI data for the sample magnetization at multiple K-space positions along a K-space trajectory and in the multi-shot readout phase.

In an example, a MRI device includes a RF transmitter, a RF receiver, a gradient system, and at least one processor. The at least one processor is configured to perform the following: in a preparation phase: controlling the RF transmitter to apply an inversion pulse to a sample magnetization; in a multi-shot readout phase: controlling the gradient system to apply a steady-state gradient echo readout sequence including at least one first phase-encoding gradient along a first direction, at least one second phase-encoding gradient along a second direction, and a sequence of readout gradients along a readout direction; and in the multi-shot readout phase: controlling the gradient system to apply first AC gradients along the first direction and at least partly contemporaneously with readout gradients of the sequence of readout gradients; in the multi-shot readout phase: controlling the gradient system to apply second AC gradients along the second direction and at least partly contemporaneously with the readout gradients of the sequence of readout gradients; and in the multi-shot readout phase: controlling the RF receiver to acquire MRI data for the sample magnetization at multiple K-space positions along a K-space trajectory.

In an example, a method includes controlling a RF transmitter of a MRI device to apply an inversion pulse to the sample magnetization in a preparation phase. The method further includes controlling a gradient system of the MRI device to apply a readout sequence in a multi-shot readout phase. The readout sequence includes at least one first phase-encoding gradient along the first direction and at least one second phase-encoding gradient along a second direction. The method further includes controlling a RF receiver of the MRI device to acquire MRI data for the sample magnetization at multiple K-space positions along a K-space trajectory in the multi-shot readout phase. Subsequent K-space positions along the K-space trajectory are offset from each other in the first direction and in the second direction.

In an example, a computer program product including program code is provided. The program code may be executed by at least one processor. Executing the program code can cause the at least one processor to perform a method. The method includes controlling a RF transmitter of a MRI device to apply an inversion pulse to the sample magnetization in a preparation phase. The method further includes controlling a gradient system of the MRI device to apply a readout sequence in a multi-shot readout phase. The readout sequence includes at least one first phase-encoding gradient along the first direction and at least one second phase-encoding gradient along a second direction. The method further includes controlling a RF receiver of the MRI device to acquire MRI data for the sample magnetization at multiple K-space positions along a K-space trajectory in the multi-shot readout phase. Subsequent K-space positions along the K-space trajectory are offset from each other in the first direction and in the second direction.

In an example, a computer program including program code is provided. The program code may be executed by at least one processor. Executing the program code can cause the at least one processor to perform a method. The method includes controlling a RF transmitter of a MRI device to apply an inversion pulse to the sample magnetization in a preparation phase. The method further includes controlling a gradient system of the MRI device to apply a readout sequence in a multi-shot readout phase. The readout sequence includes at least one first phase-encoding gradient along the first direction and at least one second phase-encoding gradient along a second direction . The method further includes controlling a RF receiver of the MRI device to acquire MRI data for the sample magnetization at multiple K-space positions along a K-space trajectory in the multi-shot readout phase. Subsequent K-space positions along the K-space trajectory are offset from each other in the first direction and in the second direction.

In an example, a MRI device includes a RF transmitter, a RF receiver, a gradient system, and at least one processor. The at least one processor is configured to perform the following: in a preparation phase: controlling a RF transmitter of a MRI device to apply an inversion pulse to a sample magnetization; and in a multi-shot readout phase: controlling a gradient system of the MRI device to apply a readout sequence including at least one first phase-encoding gradient along a first direction and at least one second phase-encoding gradient along a second direction; and in the multi-shot readout phase: controlling a RF receiver of the MRI device to acquire MRI data for the sample magnetization at multiple K-space positions along a K-space trajectory. Subsequent K-space positions along the K-space trajectory are offset from each other in the first direction and in the second direction.

In an example, a method includes controlling a MRI device to apply a 3-D MP RAGE MRI sequence using WAVE-CAIPIRINHA during a readout event.

In an example, a computer program product including program code is provided. The program code may be executed by at least one processor. Executing the program code can cause the at least one processor to perform a method. The method includes controlling a MRI device to apply a 3-D MP RAGE MRI sequence using WAVE-CAIPIRINHA during a readout event.

In an example, a computer program including program code is provided. The program code may be executed by at least one processor. Executing the program code can cause the at least one processor to perform a method. The method includes controlling a MRI device to apply a 3-D MP RAGE MRI sequence using WAVE-CAIPIRINHA during a readout event.

In an example, a method includes retrieving a coil sensitivity map for at least some coils of a RF coil assembly of an MRI device. The method further includes retrieving constraints for at least some of a plurality of scan parameters of an MRI scan employing a PAT. The method further includes predicting, based on the constraints and further based on the coil sensitivity map, SNR characteristics of the MRI scan for candidate values of the plurality of scan parameters. The method further includes selecting values of the plurality of scan parameters from the candidate values based on the SNR characteristics and controlling the MRI device to perform the MRI scan based on the selected values.

In an example, a computer program product including program code is provided. The program code may be executed by at least one processor. Executing the program code can cause the at least one processor to perform a method. The method includes retrieving a coil sensitivity map for at least some coils of a RF coil assembly of an MRI device. The method further includes retrieving constraints for at least some of a plurality of scan parameters of an MRI scan employing a PAT. The method further includes predicting, based on the constraints and further based on the coil sensitivity map, SNR characteristics of the MRI scan for candidate values of the plurality of scan parameters. The method further includes selecting values of the plurality of scan parameters from the candidate values based on the SNR characteristics and controlling the MRI device to perform the MRI scan based on the selected values.

In an example, a computer program including program code is provided. The program code may be executed by at least one processor. Executing the program code can cause the at least one processor to perform a method. The method includes retrieving a coil sensitivity map for at least some coils of a RF coil assembly of an MRI device. The method further includes retrieving constraints for at least some of a plurality of scan parameters of an MRI scan employing a PAT. The method further includes predicting, based on the constraints and further based on the coil sensitivity map, SNR characteristics of the MRI scan for candidate values of the plurality of scan parameters. The method further includes selecting values of the plurality of scan parameters from the candidate values based on the SNR characteristics and controlling the MRI device to perform the MRI scan based on the selected values.

In an example, a device includes at least one processor. The at least one processor is configured to perform the following: retrieving a coil sensitivity map for at least some coils of a RF coil assembly of the MRI device; and retrieving constraints for at least some of a plurality of scan parameters of an MRI scan employing a PAT; and based on the constraints and further based on the coil sensitivity map: predicting SNR characteristics of the MRI scan for candidate values of the plurality of scan parameters; and selecting values of the plurality of scan parameters from the candidate values based on the SNR characteristics; and controlling the MRI device to perform the MRI scan based on the selected values.

It is to be understood that the features mentioned above and those yet to be explained below may be used not only in the respective combinations indicated, but also in other combinations or in isolation without departing from the scope of the invention.

### BRIEF DESCRIPTION OF THE DRAWINGS

FIG. 1 schematically illustrates an MRI device according to various examples.
FIG. 2 is a flowchart of a method according to various examples.
FIG. 3 is a schematic pulse diagram of an MRI sequence according to various examples.
FIG. 4 is a schematic pulse diagram of an MRI sequence according to various examples, wherein the MRI sequence includes AC gradients applied contemporaneously with readout gradients along two phase-encoding directions.
FIG. 5 illustrates the AC gradients in greater detail.
FIG. 6 illustrates full sampling of the K-space according to various examples.
FIG. 7 illustrates undersampling of the K-space according to various examples.
FIG. 8 illustrates undersampling of the K-space according to a 2-D CAIPIRINHA sampling scheme and according to various examples.
FIG. 9 illustrates undersampling of the K-space according to a 2-D Wave-CAIPI sampling scheme and according to various examples.
FIG. 10 illustrates a K-space trajectory according to various examples.
FIG. 11 illustrates a zigzag-shaped K-space trajectory according to various examples.
FIG. 12 is a schematic pulse diagram of an MRI sequence according to various examples, wherein the MRI sequence includes AC gradients applied contemporaneously with readout gradients along two phase-encoding directions.
FIGs. 13 - 16 illustrate gray matter-segmented images obtained using MRI sequences according to various examples.
FIG. 17 is a flowchart of a method according to various examples, wherein FIG. 17 illustrates selection of values of scan parameters for a MRI scan.
FIG. 18 is a flowchart of a method according to various examples.
FIG. 19 is a flowchart of a method according to various examples.
FIG. 20 is a flowchart of a method according to various examples.

### DETAILED DESCRIPTION OF EMBODIMENTS

In the following, embodiments of the invention will be described in detail with reference to the accompanying drawings. It is to be understood that the following description of embodiments is not to be taken in a limiting sense. The scope of the invention is not intended to be limited by the embodiments described hereinafter or by the drawings, which are taken to be illustrative only.

The drawings are to be regarded as being schematic representations and elements illustrated in the drawings are not necessarily shown to scale. Rather, the various elements are represented such that their function and general purpose become apparent to a person skilled in the art. Any connection or coupling between functional blocks, devices, components, or other physical or functional units shown in the drawings or described herein may also be implemented by an indirect connection or coupling. A coupling between components may also be established over a wireless connection. Functional blocks may be implemented in hardware, firmware, software, or a combination thereof.

Hereinafter, techniques of MRI are described. MRI may be employed to obtain MRI data of the magnetization of a sample region of a patient. From this, images of the patient may be derived. According to some of the examples described herein, MRI techniques are provided which enable to obtain the MRI data comparably fast and with a high SNR ratio.

In some examples, MRI sequences are described. The MRI sequences describe the temporal alignment of various physical quantities to manipulate the magnetization, e.g., RF pulses and gradients. It is possible that the MRI sequences include an initial RF inversion pulse to prepare the magnetization for subsequent readout. Phase-encoding gradients can be applied along one or more phase-encoding directions to address a certain k-space position. In readout, readout gradients are applied along a readout direction; this yields MRI data lines along the readout direction. The raw MRI data is defined in K-space, i.e., spatial-frequency domain. Different echoes of the magnetization - e.g., gradient or spin echoes - may encode different positions in K-space, i.e., may help to obtain spatially-resolved MRI images. By such means, it is possible to provide images of the patient having a unique contrast. For example, the contrast may facilitate imaging of the brain of the patient. Examples include T1-dependent contrast and T2-dependent contrast.

The techniques described herein may be employed for three-dimensional (3-D) MRI sequences. Sometimes, 3-D MRI sequences are also referred to as multi-slab sequences, because MRI data for more than a single slice or slab is acquired per excitation or preparation of the magnetization. A 3-D MRI sequence may be a sequence which encodes the K-space as a 3-D space by using three nonlinear encoding axes. In contrast, 2-D MRI sequences encode K-space as multiple two dimensional encoded spaces using two nonlinear encoding axes.
3-D MRI sequences typically employ phase encoding of the MRI data along more than a single direction. Typically, phase encoding of the MRI data is employed along two phase-encoding directions that may be orthogonal. Generally, for 3-D MRI sequences, slice-selective excitation of the MRI data may be employed by applying a gradient field along a slice-selection direction; typically, one of the phase-encoding directions is then co-linear with a slice-selection direction. In other examples, it is also possible that 3-D MRI sequences are combined with 3-D excitation pulses which excite the magnetization in a region of interest having three-dimensional extents; here, time-varying gradients may be applied along multiple orthogonal directions and an amplitude modulation of the 3-D excitation pulse can be used.

According to various examples, MRI sequences using parallel acquisition techniques (PAT) may be employed. PATs typically rely on undersampling of K-space; i.e., for certain K-space positions, MRI data is not acquired and the missing information is reconstructed later on. The so-called acceleration factor R is indicative of the fraction of those K-space position along a K-space trajectory for which no MRI data is acquired. Larger (smaller) acceleration factors correspond to a shorter (longer) scan times. For reconstruction of the missing information, respectively for determining reconstructed MRI data, often a predetermined or calibrated sensitivity profile of multiple receiver coils of the RF receiver of the MRI device is used; thereby, aliasing effects resulting from the undersampling can be mitigated or removed. Examples of PAT include: Generalized AutoCalibrating Partially Parallel Acquisition (GRAPPA), see M. A. Griswold et al., in Magn. Reson. Med. 47 (2002), p. 1202 - 1210; and Sensitivity Encoding (SENSE), see K. P. Pruessmann in Magn. Reson. Med. 42 (1999), p.952 - 962; and Simultaneous Acquisition of Spatial Harmonics (SMASH), see D. K. Sodickson und W. J. Manning in Magn. Reson. Med 38 (1997), p. 591 - 603; and Breuer, Felix A., et al. "Controlled aliasing in volumetric parallel imaging (2D CAIPIRINHA)." Magnetic resonance in medicine 55.3 (2006): 549-556.

For example, conventional 2D-CAIPIRINHA generally shortens the scan time by reducing the number of phase encoding steps. This poses an intrinsic √*R* penalty on the SNR where R is the acceleration factor. Moreover, SNR is also affected by the encoding power of the PAT, also referred to as the geometry factor (g-factor). At high acceleration (approx. R>4), conventional 2D-CAIPIRINHA sometimes lacks sufficient encoding capability and localized g-factor hotspots arise, which cause severe noise amplification in the image and reduce the SNR.

An example MRI sequence on which certain implementations described herein may be based is the 3-D MP RAGE MRI sequence. Here, an inversion pulse - defining a preparation phase - such as a 180° pulse is followed by a readout phase. In the readout phase, multiple gradient echoes may be formed by a gradient pulse train. Each one of the multiple gradient echoes may be measured to yield MRI data; each gradient echo defines a readout event. Because multiple gradient echoes are formed in the context of a single preparation of the magnetization - e.g., the inversion pulse - the readout phase is also sometimes referred to as multi-shot readout phase. Subsequent RF excitation pulses can be used during the multi-shot readout phase in order to excite transversal magnetization multiple times. Because transversal magnetization is rapidly excited during the gradient pulse train, a steady-state of the transversal magnetization results. Because of this, the gradient echo readout sequence is sometimes referred to as steady-state gradient echo readout sequence. The preparation phase and the readout phase are typically repeated multiple times; each repetition defines an iteration. Different iterations can be separated by a relaxation phase which allows to the magnetization to return to its relaxation position aligned with a DC magnetic field. Different iterations are used to acquire MRI data across the entire K-space. The iterations of preparation phase - readout phase - and relaxation phase define an outer loop of the 3-D MP RAGE MRI sequence. The multiple readout events per readout phase define an inner loop of the 3-D MP RAGE MRI sequence.

In one example, 3-D MP RAGE is enhanced based on techniques of Wave-CAIPI. See Bilgic, Berkin, et al. "Wave-CAIPI for highly accelerated 3-D imaging." Magnetic resonance in medicine 73.6 (2015): 2152-2162 which is incorporated herein in its entirety by reference. Wave-CAIPI modifies the conventional gradient echo readout phase by applying AC gradient pulses - e.g, sinusoidal waveforms - on the phase-encoding directions during the sampling period, i.e., the readout events. It is thus possible to apply first AC gradients along a first phase-encoding direction and second AC gradients along a second phase-encoding direction at least partly contemporaneously with readout gradients. Generally, due to imperfections of the gradient system, the actual AC gradients can vary significantly from the nominal waveforms used for the gradient pulses; this may be accounted for by appropriate calibration techniques or compensation techniques during reconstruction. The AC gradients provide voxel spreading in the readout direction. As the amount of voxel spreading is dependent on the K-space position along the phase-encoding directions, such wave encoding typically improves the coil sensitivity variation in the collapsed voxels for PATs. This results in an increased SNR, because reconstruction techniques of parallel imaging can more precisely anti-alias the MRI image data.

In a further example, 3-D MP RAGE may be enhanced based on a reordering scheme for implementing a tailored K-space trajectory when acquiring MRI data along multiple phase-encoding directions. Here, a K-space trajectory which has non-zero dimensions in both phase-encoding directions for each repetition can be employed. For example, subsequent K-space positions along the K-space trajectory may be offset from each other in both, the first phase-encoding direction and the second phase-encoding direction.

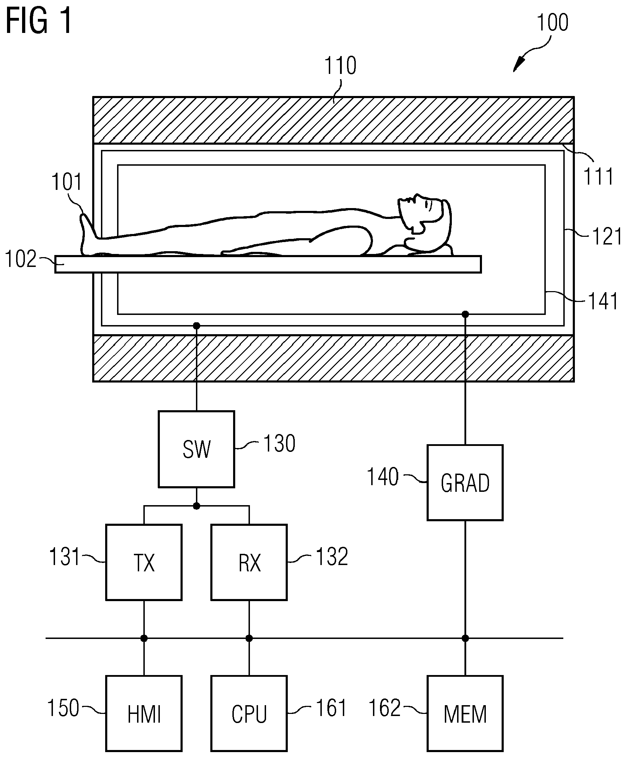

FIG. 1 illustrates aspects with respect to an MRI device 100. The MRI device 100 includes a magnet 110 which defines a bore 111. The magnet 110 can provide a DC magnetic field of one to six Tesla along its longitudinal axis. The DC magnetic field can align the magnetization of the patient 101 along the longitudinal axis. The patient 101 can be moved into the bore by means of a movable table 102.

The MRI device 100 also includes a gradient system 140 for creating spatially-varying magnetic gradient fields (gradients) used for spatially encoding MRI data. Typically, the gradient system 140 includes at least three gradient coils 141 that are arranged orthogonal to each other and can be controlled individually. By applying gradient pulses to the gradient coils 141, it is possible to apply gradients along certain directions. The gradients can be used for slice selection (slice-selection gradients), frequency encoding (readout gradients), and phase encoding along one or more phase-encoding directions (phase-encoding gradients). Hereinafter, the slice-selection direction will be defined as being aligned along the Z-axis; the readout direction will be defined as being aligned with the X-axis; and a first phase-encoding direction as being aligned with the Y-axis. A second phase-encoding direction may be aligned with the Z-axis. The directions along which the various gradients are applied are not necessarily in parallel with the axes defined by the coils 141. Rather, it is possible that these directions are defined by a certain K-space trajectory which, in turn, can be defined by certain requirements of the respective MRI sequence and/or based on anatomic properties of the patient 101.

For preparation and/or excitation of the magnetization polarized/aligned with the DC magnetic field, RF pulses can be applied. For this, and RF coil assembly 121 is provided which is capable of applying an RF pulse such as an inversion pulse or an excitation pulse. While the inversion pulse generally inverts the direction of the longitudinal magnetization, excitation pulses can create transversal magnetization.
For creating such RF pulses, a RF transmitter 131 is connected via a RF switch 130 with the coil assembly 121. Via a RF receiver 132, it is possible to detect signals of the magnetization relaxing back into the relaxation position aligned with the DC magnetic field. In particular, it is possible to detect echoes; echoes may be formed by applying one or more RF pulses (spin echo) and/or by applying one or more gradients (gradient echo). The magnetization may inductively coupled with the coil assembly 121 for this purpose. The respectively acquired MRI data can correspond to raw data in K-space; according to various examples, the MRI data can be postprocessed in order to obtain images. Such postprocessing can include a Fourier Transform from K-space to image space. Such postprocessing can also include reconstruction to avoid aliasing where the K-space is undersampled according to a PAT.

Generally, it would be possible to use separate coil assemblies for applying RF pulses on the one hand side and for acquiring MRI data on the other hand side (not shown in FIG. 1). For example, for applying RF pulses a comparably large body coil 121 can be used; while for acquiring MRI data a surface coil assembly including an array of comparably small coils could be used. For example, the surface coil assembly could include 32 individual RF coils and thereby facilitate PATs relying on spatially-offset coil sensitivities.

The MRI device 100 further includes a human machine interface 150, e.g., a screen, a keyboard, a mouse, etc. By means of the human machine interface 150, a user input can be detected and output to the user can be implemented. For example, by means of the human machine interface 150, it is possible to set certain configuration parameters for the MRI sequences to be applied.

The MRI device 100 further includes a processor 161. The processor 161 may implement various control functionality with respect to the operation of the MRI device 100. For example, the processor 161 could implement a sequence control for time-synchronized operation of the gradient system 140, the RF transmitter 131, and the RF receiver 132. For example, the processor 161 could implement postprocessing of acquired MRI data. In some examples, instead of using a single processor 161, multiple processors could be used.

For implementing the respective functionality, the processor 161 may retrieve program code from the memory 161, e.g., a non-volatile memory. Execution of the program code by the processor 161 can cause the processor to perform the various techniques of sequence control and image postprocessing as described herein.

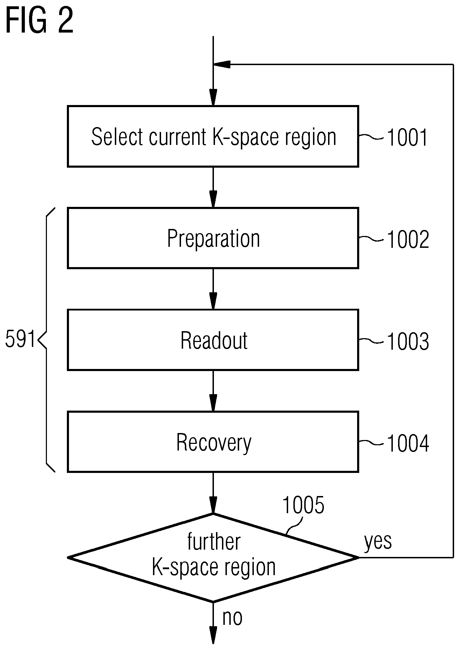

FIG. 2 is a flowchart of a method according to various examples. FIG. 2 also illustrates aspects with respect to an example MRI sequence. For example, the method according to FIG. 2 could be executed by the processor 160 of the MRI device 100.

First, in block 1001, a K-space region is selected from a plurality of K-space regions. For example, the plurality of K-space regions may define a region of interest of the patient 101 for which images should be provided. As such, the current K-space region selected in block 1001 can correspond to a subfraction of the entire region of interest. For example, in block 1001 it would be possible to determine a start position of a K-space trajectory, the start position being defined with respect to at least one phase-encoding direction.

Then, in block 1002, a preparation phase to prepare the magnetization in the selected K-space region is performed. For example, the preparation phase may include applying an inversion pulse to the magnetization. The inversion pulse may cause antiparallel alignment of the magnetization with the DC magnetic field. For example, the inversion pulse may be a 180° pulse. For example, the inversion may cause antiparallel alignment of the magnetization with the DC magnetic field. If the Z-axis is aligned with the DC magnetic field, this may correspond to a mirroring of the magnetization at the origin of the K-space along the Z-axis.

Then, relaxation of the magnetization into its relaxation position having parallel alignment with the DC magnetic field occurs; after the so-called inversion time (TI), the magnetization has halfway relaxed into the relaxation position and may have a zero component along the Z-axis.

In block 1003, a readout phase is performed. Here, a multi-shot acquisition may be performed where MRI data for multiple K-space positions is acquired: This includes acquiring MRI data for multiple K-space positions of the magnetization prepared in block 1002. Because in block 1003 MRI data for multiple K-space position is acquired, block 1003 is sometimes referred to as inner loop. Here, different iterations of applying RF pulses and/or readout gradients can be used.

According to examples, in block 1003, phase-encoding gradients are applied along two orthogonal phase-encoding directions.

Various readout sequences may be applied in block 1003. Example is include a steady-state gradient echo readout sequence, e.g., as described in Haase, Axel. "Snapshot flash mri applications to t1, t2, and chemical-shift imaging." Magnetic Resonance in Medicine 13.1 (1990): 77-89. Further examples include 3-D Fluid-Attenuated Inversion Recovery (3-D FLAIR), see for example Naganawa, Shinji, et al. "Comparison of flow artifacts between 2-D-FLAIR and 3-D-FLAIR sequences at 3 T." European radiology 14.10 (2004): 1901-1908. Still further examples include 3-D SPACE MRI sequences, see Lichy, Matthias Philipp, et al. "MRI of the body trunk using a single-slab, 3-dimensional, T2-weighted turbo-spin-echo sequence with high sampling efficiency (SPACE) for high spatial resolution imaging: initial clinical experiences." Investigative radiology 40.12 (2005): 754-760.

In block 1004, a relaxation phase is employed. During the relaxation phase, magnetization may recover from an excited state into the relaxation state aligned with the DC magnetic field.

In block 1005 it is checked whether MRI data for a further K-space region is required. For example, in block 1005 it may be checked whether sufficient MRI data has been acquired in previous iterations of 1001 - 1004 in order to reconstruct an image of the entire region of interest. If, at block 1005, it is judged that further MRI data is required, a further iteration 591 of blocks 1001 - 1004 may be performed using at least partly different phase-encoding gradients than for the previous iteration. This defines an outer loop of the MRI sequence. However, if it is judged that sufficient MRI data has been acquired, the method may commence with postprocessing (not illustrated in FIG. 2).

The techniques illustrated in FIG. 2 may be combined with PATs. In one example, a so-called outer-loop PAT may be employed. Here, the outer loop defined by blocks 1001 and 1005 of the MRI sequence may be configured to undersample the K-space. For example, it could be possible that in block 1001 a certain K-space region is selected by a specific position along the Y-axis. Then, it could be possible to select the K-space regions in block 1001 by skipping certain positions along the Y-axis. Outer loop PAT generally reduces the number of iterations 591 and hence reduces the overall scan time.

Outer loop PATs may be employed alternatively or additionally with respect to inner-loop PATs. For inner loop PATs, it is possible to undersample the K-space when performing the readout phase 1003. For example, it could be possible that in block 1003 phase encoding of the magnetization is employed along two phase-encoding directions, e.g., aligned with the Y-axis and the Z-axis. Then, certain K-space positions could be skipped in block 1003 by appropriately configuring the phase-encoding gradients.

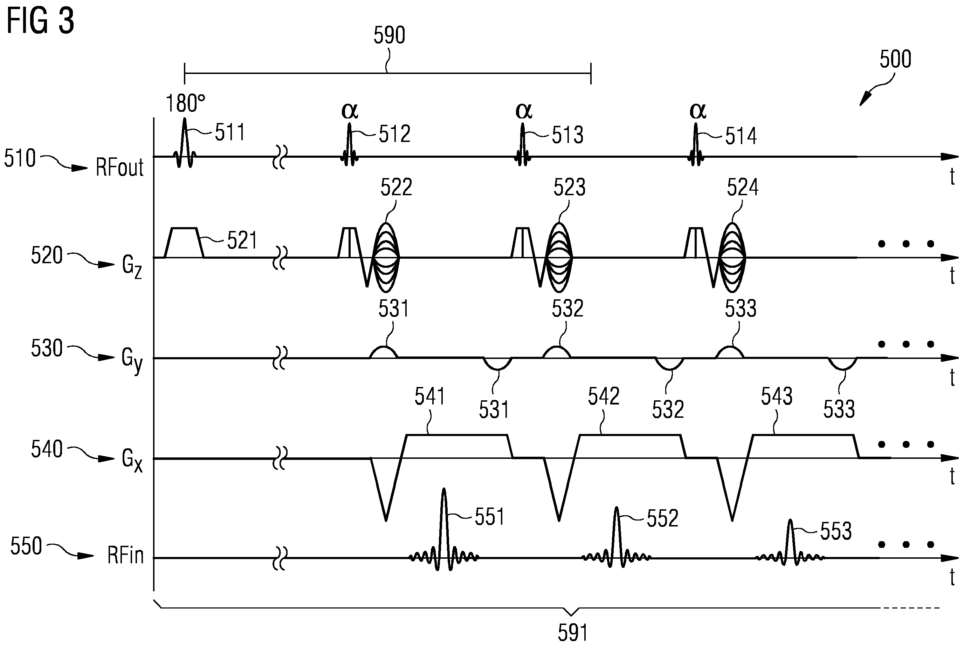

FIG. 3 illustrates aspects with respect to an example MRI sequence 500. FIG. 3 is a pulse diagram of the example MRI sequence 500. FIG. 3 illustrates the inner loop of block 1003 according to the flowchart of FIG. 2. In FIG. 3, aspects with respect to a 3-D MP RAGE MRI sequence 500 are illustrated.

First, a RF inversion pulse 511 is applied to the magnetization (shown for RF send channel 510). In the example of FIG. 3, the inversion pulse 511 is slice selective and, hence, accompanied by a gradient 521 applied along the slice-selection direction 520. This defines the preparation phase. After some time, a sequence of excitation pulses 512 - 514 labeled α in FIG. 3 is applied. Each excitation pulse 512 - 514 defines a readout event. While in the example of FIG. 3 a sequence of three pulses as illustrated, generally, a larger number of pulses could be applied to implement more readout events. For example, a number of 5 - 30 pulses could be applied for each iteration of the inner loop. The pulses are excitation pulses having a flip angle of preferably less than 90°, e.g., in the range of 10 - 40°. The excitation pulses flip a fraction of the longitudinal magnetization into the transversal plane. The excitation pulses 512-514 are closely packed in time domain so that the magnetization has no time to recover and hence a steady state of the magnetization evolves. Thus, the sequence of excitation pulses 512-514 defines a steady-state readout sequence.

Each excitation pulse 512 - 514 or readout event defines an iteration of the inner loop. In particular, each excitation pulse 512 - 514 is associated with the respective echo 551 - 553 of the magnetization (shown for RF receive channel 550). The echoes 551 - 553 are gradient echoes formed by gradients 541 - 543 applied along the readout direction 540.

FIG. 3 illustrates a 3-D MRI sequence 500 including phase encoding along two phase-encoding directions 520, 530; in the example of FIG. 3, the two phase-encoding directions 520, 530 are orthogonal to each other, but may generally be linearly independent of each other. Phase-encoding gradients 522 - 524 are applied on channel 520 along the phase-encoding direction 520. For different iterations of the inner loop, different amplitudes are used for the phase-encoding gradients 522 - 524 to move throughout the K-space; thereby, a K-space trajectory visiting different K-space positions is implemented. In the example of the MRI sequence 500 of FIG. 3, phase-encoding gradients 531 - 533 are applied along the phase-encoding direction 530. In the example of FIG. 3, the prewinder and rewinder gradients 531 - 533 all have the same amplitude, respectively. Hence, the K-space trajectory thereby defined does not visit K-space positions having different positions along the phase-encoding direction 530

In FIG. 3, the TI 590 is illustrated. As is apparent, the echoes 551 - 553 are approximately centered with respect to the TI 590. Thereby, the T1-contrast in the images obtained from the MRI data can be increased. In particular, it can be desirable to acquire MRI data close to the K-space center at times that are adjacent to the TI 590 (for example, the echo 552 could correspond to a K-space position at z=0).

It has been observed that the 3-D MRI sequence 500 according to the example of FIG. 3 can suffer from reduced SNR ratio, in particular, if inner loop PATs for acceleration are employed.

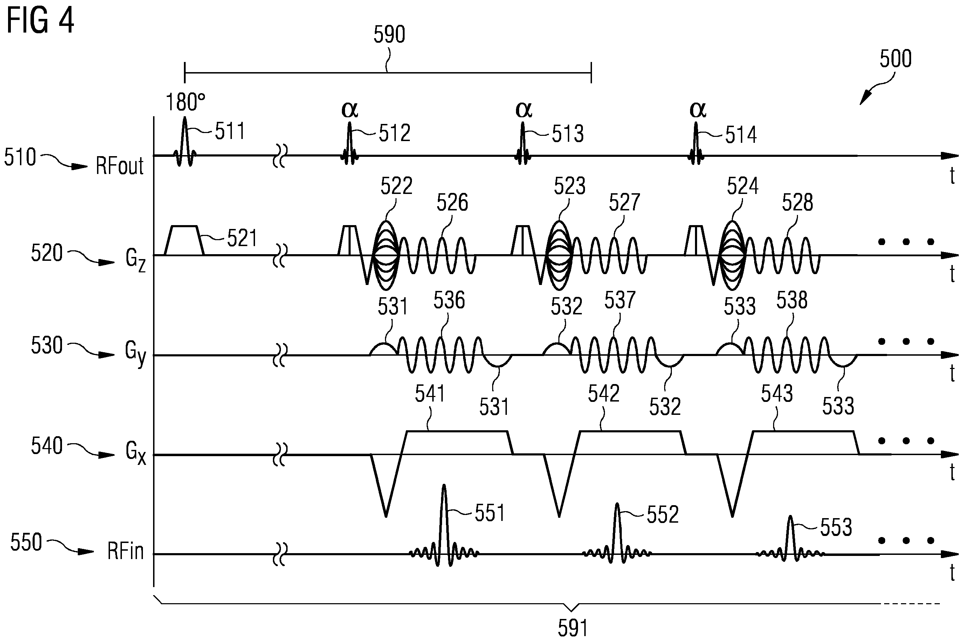

To solve this issue, according to various examples, the 3-D MP RAGE MRI sequence 500 according to the example of FIG. 3 can be combined with Wave-CAIPI during the readout phase. This is illustrated in FIG. 4.

FIG. 4 illustrates aspects with respect to an example MRI sequence 500. FIG. 4 is a pulse diagram of the example MRI sequence 500. The example MRI sequence 500 of FIG. 4 generally corresponds to the example MRI sequence 500 according to FIG. 3.

Additionally, in the example of FIG. 4, AC gradients 526 - 528 are applied along the phase-encoding direction 520 and AC-gradients 536 - 538 are applied along the phase-encoding direction 530. In particular, the AC gradients 526 - 528, 536 - 538 are applied contemporaneously with the readout gradients 541 - 543; as such, the AC gradients 526 - 528, 536 - 538 are applied during readout events of the MRI data on the RF receive channel 550.

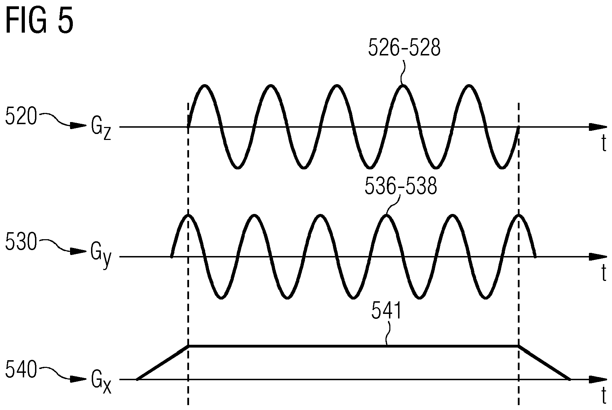

FIG. 5 is a detailed view of the AC gradients 526 - 528, 536 - 538. As illustrated in FIG. 5, the AC gradients 526 - 528, 536 - 538 are applied substantially during the entire flat top of the readout gradient 541. Generally, the AC gradients 526 - 528, 536 - 538 may be applied at least partly contemporaneously with the readout gradients 541-543.

For example, the various AC gradients 526 - 528, 536 - 538 may all have the same frequency. The frequency may be in the range of 50 Hz - 50 kHz. For example, this may correspond to applying between 2 and 20 periods of the AC gradients 526 - 528, 536 - 538 per readout gradient 541 - 543. In some examples, the AC gradients 526 - 528, 536 - 538 may be described by a sinusoidal function. It is possible that the AC gradients 526 - 528 applied along the phase-encoding direction 520 exhibit a phase shift if compared to the AC gradients 536 - 538 applied along the phase-encoding direction 530; e.g., the phase shift may be in the order of 5° - 90°, optionally in the order of 10 - 30°.

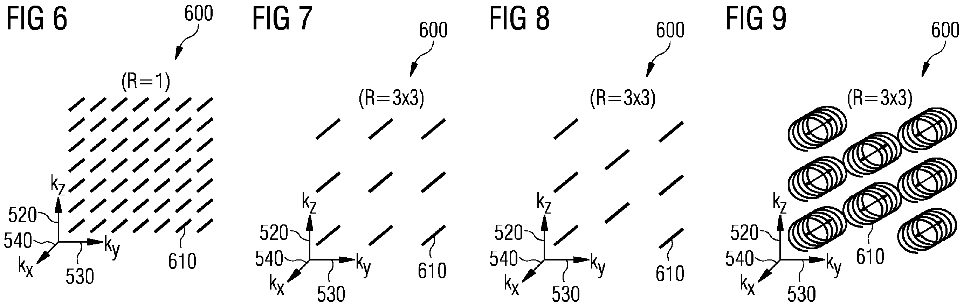

FIGs. 6 - 9 illustrated examples with respect to inner-loop PATs.

FIG. 6 illustrates non-accelerated acquisition of MRI data for various K-space positions 610. FIG. 6 is a perspective view. In the example of FIG. 1, R=1. The K-space is fully sampled to avoid aliasing. For each K-space position in the plane defined by the Z-axis and the Y-axis - i.e., the phase-encoding directions 520, 530 -, MRI data is acquired along a respective line co-linear with the X-axis - i.e., the readout direction 540.

FIG. 7 generally corresponds to the example of FIG. 6. However, in the example of FIG. 7 the K-space 600 is undersampled. FIG. 7 illustrates inner-loop acceleration; hence, the K-space 600 is undersampled in a plane defined by the phase-encoding directions 520, 530. The acceleration factor is *R=3* in each phase-encoding direction, because along each phase-encoding direction only for every third K-space position 610 MRI data is acquired. Such undersampling enables to take MRI data for a larger K-space region per time interval.

For example, the image reconstruction for such an undersampled K-space 600 could be employed using GRAPPA techniques. This is because the K-space position 610 for which MRI data is acquired are arranged on a pattern having a rectangular or square unit cell.

FIG. 8 generally corresponds to the example of FIG. 7. However, in the example of FIG. 8, the K-space 600 is undersampled using a different pattern, i.e., a 2-D CAIPIRINHA pattern. Here, nearest-neighbor K-space positions for which MRI data are obtained are offset from each other in both phase-encoding directions. This increases the SNR when reconstructing missing MRI data; the g-factor penalty is reduced.

FIG. 9 generally corresponds to the example of FIG. 8. FIG. 9 illustrates aspects with respect to applying the AC gradients along the phase-encoding directions 520, 530 and contemporaneously with the readout gradients. In FIG. 9, the corkscrew-shaped readout lines obtained from the AC gradients are illustrated. This is sometimes referred to as voxel spreading. This further increases the SNR when reconstructing missing MRI data; the g-factor penalty is reduced.

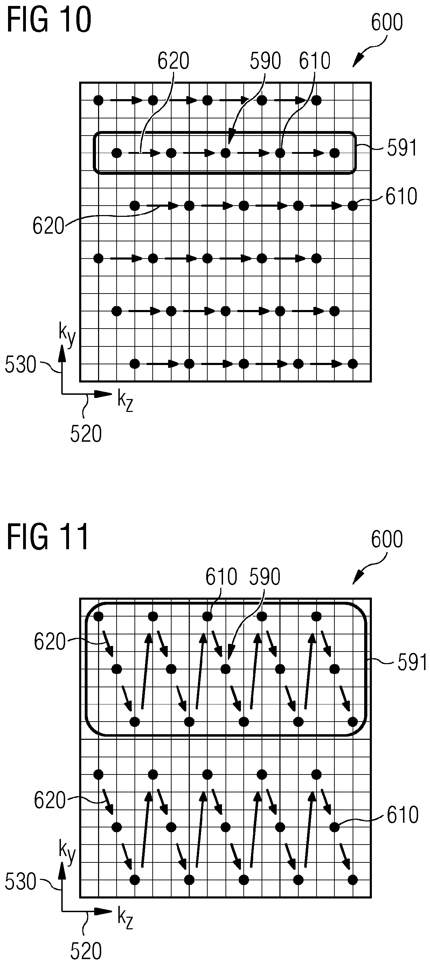

FIG. 10 illustrates aspects with respect to a K-space trajectory 620. FIG. 10 illustrates the K-space trajectory 620 for the MRI sequence 500 according to the example of FIGs. 3 and 4 (in FIG. 10, the corkscrew-shaped readout lines due to AC gradients are not illustrated for sake of simplicity). A CAIPIRINHA pattern is used (cf. FIGs. 8 and 9) .

In FIG. 10, the K-space positions 610 along the K-space trajectory 612 for a given iteration 591 of the outer loop of the MRI sequence 500 are highlighted. Here, it is apparent that subsequent K-space positions 610 along the K-space trajectory are offset from each other only along the phase-encoding direction 520; subsequent K-space positions 610 along the K-space trajectory are not offset from each other along the phase-encoding direction 530. Thus, a linear K-space trajectory 620 is obtained for each iteration 591.

In FIG. 10, the K-space trajectory 620 - for each iteration 591 - includes K-space positions 610 having, both, positive K values and negative K values along the first phase-encoding direction 520, i.e., the Z-axis. Thereby, it is possible to acquire MRI data at a K-space position 610 close to the center of the K-space is approximately the TI 590. This increases the T1-contrast of the image.

FIG. 11 illustrates aspects with respect to a K-space trajectory 620. In FIG. 11 - differently from the scenario of FIG. 10 - adjacent K-space positions along the K-space trajectory 620 are offset from each other in, both, the phase-encoding direction 520 and the phase-encoding direction 530. Generally, any two subsequent K-space positions could be offset in both direction 520, 530.

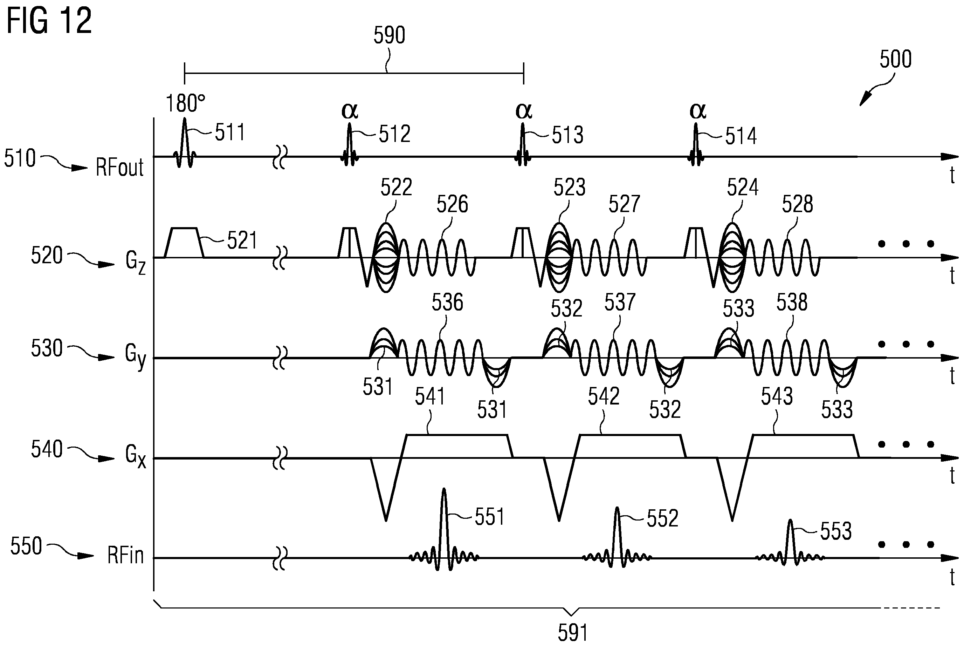

An example MRI sequence 500 that could be used for implementing the K-space trajectory 620 according to the example of FIG. 11 is illustrated in FIG. 12.

FIG. 12 illustrates aspects with respect to an MRI sequence 500. The MRI sequence 500 according to the example of FIG. 12 generally corresponds to the MRI sequence 500 according to the example of FIG. 4. However, in the example of FIG. 12, there is also a variation of the amplitude of the phase-encoding gradients 531, 532, 533 applied along the phase-encoding direction 530. Thereby, the offset between adjacent K-space position 610 along the K-space trajectory 620 in both phase-encoding directions can be achieved.

Again referring to FIG. 11: The offset between adjacent K-space positions 610 along the K-space trajectory 620 allows to achieve the following effect: it is possible to acquire more MRI data 610 close to the TI 590; thereby, the overall contrast - e.g., T1-contrast - of the image can be increased. In other words, by offsetting between adjacent K-space positions 610 along the K-space trajectory 620, multiple planes defined by the X-axis and the Z-axis (perpendicular to the drawing plane of FIG. 11) can be acquired in an interleaved manner. The K-space center of each such plane is then acquired close to TI which provides the desired contrast.

For example, as is apparent from FIG. 11, the MRI data at a K-space position 610 of the K-space trajectory 620 corresponding to a K value of zero along the phase-encoding direction 520 aligned with the Z-axis is obtained adjacent to the TI 590; notably, this holds true for K-space positions 610 having different positions along the Y-axis.

In the example of FIG. 11, the K-space trajectory 620 is zigzag-shaped in the plane defined by the phase-encoding directions 520, 530. The zigzag-shaped trajectory is implemented by the K-space trajectory 620 cyclically visiting the same K-space positions along the phase-encoding direction 530; while continuously progressing along the phase-encoding direction 520. By implementing the zigzag-shaped K-space trajectory 620, it is possible to reduce the gradient slew rate required for the phase-encoding gradients 522 - 524, 531 - 533.

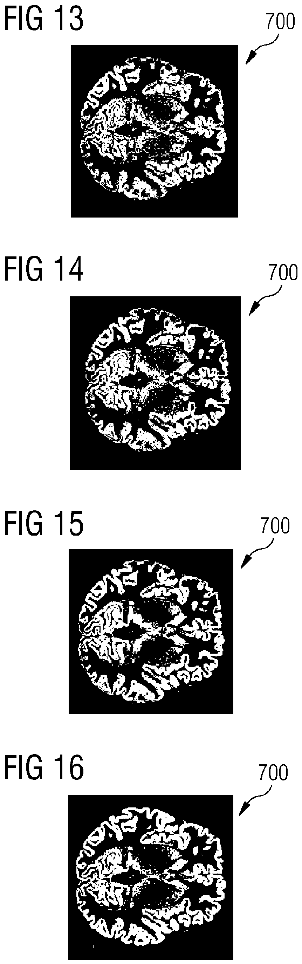

FIGs. 13 - 16 illustrate images 700 obtained from techniques as described herein. All images 700 illustrated in FIGs. 13 - 16 have been obtained using a 3-D MP RAGE MRI sequence and show the brain of a patient. FIGs. 13 - 16 have been postprocessed to segment between areas including gray matter and areas not including gray matter.

Here, FIG. 13 describes a R=3x3 2-D CAIPIRINHA inner-loop accelerated image 700 (cf. FIG. 8). FIG. 14 corresponds to FIG. 13, but also uses Wave-CAIPI, i.e., AC gradients along the phase-encoding directions during readout (cf. FIGs. 4 and 9). FIG. 15 corresponds to FIG. 14, but includes three averages per MRI data. FIG. 16 is a reference scan using R=4x1 GRAPPA inner loop acceleration.

Summarizing, above techniques have been described which enable highly accelerated 3-D MP RAGE MRI with negligible g-factor noise penalty. For this, a combination of 3-D MP RAGE with Wave-CAIPI and/or a reordered k-space zig-zag sampling scheme for in-plane acceleration is applied.

Wave-CAIPI modifies the conventional gradient echo readout by playing AC gradients along the phase-encoding directions during the readout events which produces voxel spreading in the readout direction. As the amount of voxel spreading is dependent on the spatial positions along Y-axis and Z-axis, the coil-sensitivity variation in the collapsed voxels for accelerated acquisitions is improved. This facilitates reconstruction of missing MRI data.

Above, various MRI sequences have been described which may benefit from reduced scan times. For this, PATs can be employed. Optionally, 3-D sampling of the K-space can be performed using at least two phase-encoding directions. As will be appreciated from the above, due to the complexity of the MRI sequences, a wide parameter space of available values of the scan parameters of the MRI scan results. It has been observed that due to the manifold settings available, it can sometimes be difficult to select the optimum values for the scan parameters. This may result in degraded characteristics of the MRI scan such as: reconstruction artifacts; increased scan time; reduced SNR; etc.

Various examples are described which facilitate selection of values of scan parameters of an accelerated MRI scan employing a PAT. For example, the selection may be made prior to performing the MRI sequence, e.g., using a phantom sample for characterization. The selection may alternatively be made in a planning phase of the MRI scan, e.g., when the patient 101 has been placed on the table 102 inside the bore 111 of the MRI device 100 or when the patient 101 is about to be placed on the table 102 inside the bore 111 of the MRI device 100. In particular, it is possible that the selection can be re-executed every time a new MRI scan is initiated; thereby, it can be ensured that the optimum values for the scan parameters are selected given the current requirement and temporal drifts are avoided.

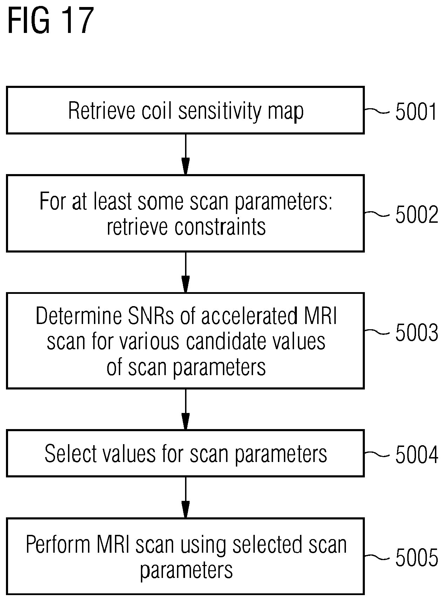

FIG. 17 is a flowchart of a method according to various examples. The method according to the example of FIG. 17 illustrates techniques with respect to selection of values of scan parameters of a MRI scan. For example, the MRI scan for which the selection of values of scan parameters is performed may be one of the MRI scans that have been described herein. Examples of the MRI scan include a 3-D MRI scan using PAT. For example, a 3-D MP RAGE MRI scan, possibly enhanced with inner-loop PAT and/or Wave CAIPI could be subject to the techniques described with respect to FIG. 17.

For example, the method according to FIG. 17 may be executed by the processor 161 of the MRI device 100. For example, the processor 161 may receive program code stored in the memory 162 and execute the program code; executing the program code can cause the processor 161 to perform the method according to the example of FIG. 17.

First, in block 5001, a coil sensitivity map is retrieved for at least some coils of the RF coil assembly 121 of the MRI device 100. The coil sensitivity map may be retrieved for the coil assembly 121 used for readout/sensing of the magnetization. In some examples, the coil sensitivity map may be predetermined and stored in the memory 162; then, the coil sensitivity map may be retrieved from the memory 162. In other examples, it is possible to perform a reference scan in order to obtain the coil sensitivity map.

In block 5002 constraints are retrieved for at least some scan parameters of the MRI sequence. For example, the constraints may correspond to acceptable values or value ranges of the at least some scan parameters. It may be possible that the constraints are defined by technological and/or physiological thresholds imposed by the MRI device 100 and/or the patient 101. Examples include a maximum slew rate of the gradients; a specific absorption rate (SAR) of RF power due to RF pulses; a breath-hold time; a maximum scan time; etc.

The constraints may be preprogrammed as control data stored in the memory 162. Alternatively or additionally, at least some of the constraints may be received from the human machine interface 150; thereby, the user may specify certain requirements for the MRI sequence.

Next, SNR characteristics of the MRI scan are predicted. This helps to obtain a-priori knowledge on the expected quality of the MRI scan. For example, different sets of candidate values for the plurality of scan parameters can be used; for each set of candidate values an associated SNR or image quality characteristic could be determined.

Here, the candidate values may be chosen in accordance with the constraints. For example, if the constraints specify a certain range within which the values of the scan parameters can be adjusted, it would be possible to choose different candidate values which are spread across said range. If, however, the constraints specify a fixed value for a given scan parameter, then a respective candidate value can be chosen which is not allowed to deviate from the fixed value. Depending on how much the various scan parameters are allowed to be varied, it can be required to predict a plurality of SNR or image quality characteristics in order to adequately sample the available parameter range.

In block 5004, preferred values of the plurality of scan parameters are selected. Here, the predicted SNR or image quality characteristics can be taken into account. For example, the preferred values may correspond to the set of candidate values having the highest associated SNR or image quality characteristic. In a further example, the preferred values of scan parameters selected in block 5004 could be those candidate values which implement a minimum scan time. It is possible to consider trade-offs between various target properties of the MRI scan when selecting the preferred values in block 5004.

In one example, it could be possible to combine blocks 5003, 5004 into an optimization. The optimization may be suited to efficiently cover the available parameter range defined by the constraints of block 5002. The optimization may further be suited to take into account trade-offs between various target properties of the MRI scan. The optimization may help to efficiently and quickly find the preferred values of the scan parameters; this may reduce the time required to plan the MRI scan.

Finally, in block 5005, the MRI scan is performed using the preferred values of the scan parameters selected in block 5004.

By such a technique it is possible to avoid low-quality MRI scans. In particular, local g-factor hotspots correspond to localized artifacts due to PAT can be identified beforehand and countermeasures can be taken.

Further, by such measures the user no longer needs to manually specify potentially suboptimal values for the scan parameters. Due to the complexity of some MRI scans, manual selection may be error prone and/or time-consuming.

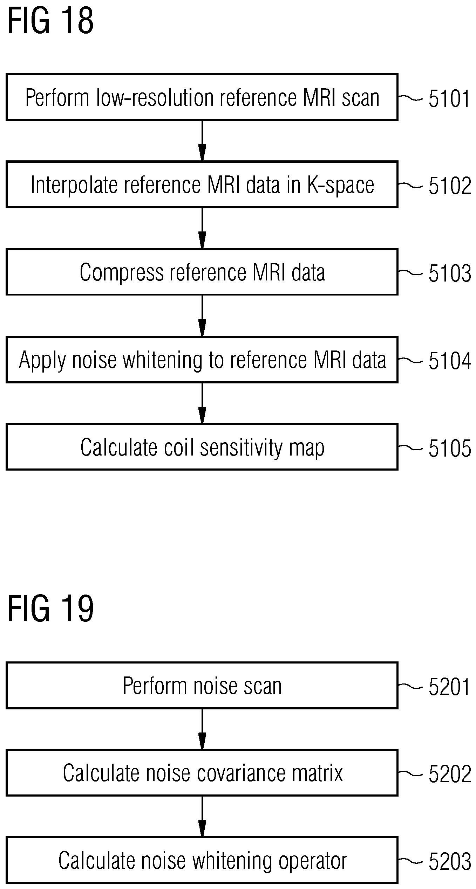

FIG. 18 is a flowchart of a method according to various examples. The method according to the example of FIG. 18 illustrates techniques with respect to retrieving the coil sensitivity map for each one of the coils of the coil assembly 121. For example, the method according to the example of FIG. 18 could be executed as part of block 5001 according to the example of FIG. 17.

In block 5101 a low-resolution reference MRI scan is performed. In particular, the low-resolution reference MRI scan of block 5101 may be performed with a resolution which is lower than the resolution of the subsequent MRI scan used to obtain the final MRI data (cf. FIG. 17: block 5005). An example resolution would be up to 50 x 50 lines, optionally up to 25 x 25 lines.

By performing a low-resolution reference MRI scan, the scan time required to obtain the respective reference MRI data can be shortened. For example, the scan time required to perform the reference MRI scan at the low resolution may be less than 1 minute. This facilitates quick planning of the MRI sequence.

For example, it would be possible that the low-resolution reference MRI scan is performed with the patient 101 already been placed inside the bore 111 on the table 102; likewise, the low-resolution reference MRI scan at block 5101 can be performed using the RF coil assembly 121 later on used to perform the final MRI scan (cf. FIG. 17: block 5005). The low-resolution reference MRI scan can image a sample volume which is at least overlapping with the sample volume of the final MRI scan (cf. FIG. 17: block 5005). As such, the MRI device 100 may be fully set up. This ensures that between the time of performing the reference MRI scan and the time of performing the final MRI scan no or only little temporal drift occurs.

In block 5102, the reference MRI data obtained from the low-resolution reference MRI scan of block 5101 is interpolated in K-space in order to increase its resolution. For example, it would be possible to increase the resolution to match the resolution of the final MRI scan (cf. FIG. 17: block 5005).

Then, at block 5103, a compression of the reference MRI data (upscaled in block 5102) is performed. The compression can help to remove ambiguous data; ambiguous data can result from multiple coils of the coil assembly 121 being arranged in close proximity to each other. For example, it could be possible to remove reference MRI data from certain coils of the coil assembly 121; such reference MRI data may be removed which does not add significant information beyond further reference MRI data of another coil, e.g., arranged in close spatial proximity. The compression may yield virtual coils; for example, the reference MRI data associated with a given virtual coil may be determined based on the measured reference MRI data associated with the plurality of coils of the coil assembly 121. By means of the virtual coils it can be possible to reduce the amount of data, but, at the same time, avoid loss of significant information. For example, it could be possible to employ a singular value decomposition in block 5103. The compression is done in order to reduce the time required to calculate the coil sensitivity map later on at block 5105.

At block 5104, noise whitening is applied to the reference MRI data. The noise whitening helps to reduce the influence of noise on the reference MRI data. Thereby, the coil sensitivity map can be calculated with enhanced accuracy in block 5105.

In block 5105, the coil sensitivity map is calculated. An example approach to do this is the eigenvalue approach of Uecker, Martin, et al. "ESPIRiT-an eigenvalue approach to autocalibrating parallel MRI: where SENSE meets GRAPPA." Magnetic resonance in medicine 71.3 (2014): 990-1001 which is incorporated herein in its entirety by reference.

The various blocks described with respect to FIG. 18 are optional. For example, instead of upscaling of the reference MRI data by means of interpolation in block 5102, it would be possible to already obtain high-resolution reference MRI data in block 5101. For example, instead of compressing the reference MRI data in block 5103, it would also be possible to calculate the coil sensitivity map in block 5105 using an uncompressed data set. Furthermore, it could be possible to not apply the noise whitening in block 5104, e.g., if appropriate noise data is not available.

FIG. 19 is a flowchart of a method according to various examples. FIG. 19 illustrates aspects with respect to calculating a noise whitening operator. For example, the noise whitening operator which is calculated according to the method of FIG. 19 may be applied in block 5104 of FIG. 18.

First, in block 5201, a noise scan is performed. The noise scan may help to quantify noise present in the system defined by the coils of the coil assembly 121, optionally already aligned with respect to the final sample volume of the MRI scan (cf. FIG. 17: block 5005). For example, the noise scan may detect a signal without previous excitation of the magnetization. Thereby, the detected signal can be attributed to background.

Based on the noise scan of block 5201, it is then possible to calculate a noise covariance matrix in block 5202. The noise covariance matrix defines the noise for the respective coils of the coil assembly 121 and additionally defines the crosstalk between pairs of coils of the coil assembly 121.

Then, based on the noise covariance matrix, it is possible to calculate the noise whitening operator in block 5203. For example, this may include an inversion of the noise covariance matrix. The noise whitening operator helps to cancel the impact of the noise from the reference MRI data.

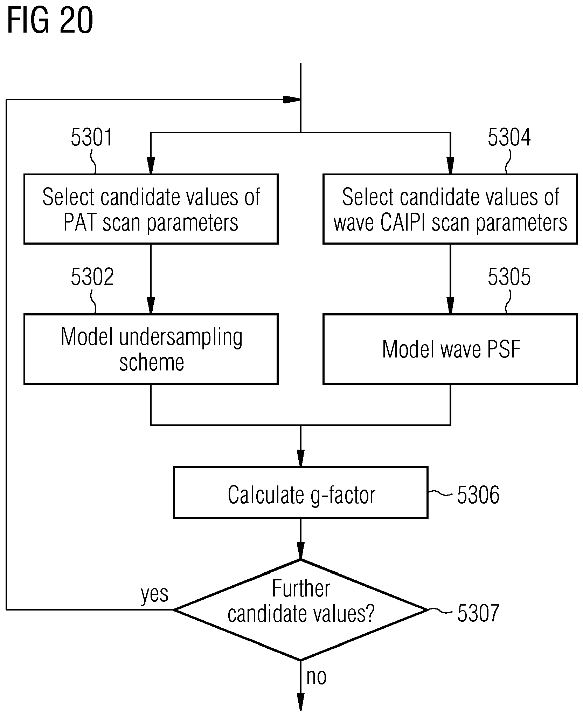

FIG. 20 is a flowchart of a method according to various examples. FIG. 20 illustrates aspects with respect to predicting the SNR characteristics of the MRI scan for various candidate scan parameters. For example, the method according to FIG. 20 may be performed as part of blocks 5003 and 5004 of FIG. 17.

FIG. 20 illustrates how different scan parameters may influence the SNR characteristic predicted for the MRI scan. Generally, the SNR characteristic may be predicted for various MRI sequences. Then, the number and type of considered scan parameters may vary with the particular MRI sequence considered. For example, if an accelerated MRI scan employing a PAT is considered, then it can be possible to consider scan parameters related to the PAT (PAT scan parameters).

Accordingly, in block 5301 candidate values of PAT scan parameters are selected. For example, it could be possible that a given PAT scan parameter relates to the acceleration factor R of the PAT, i.e., specifies the number of K-space positions left out when undersampling the K-space. Alternatively or additionally, it could be possible that a given PAT scan parameter relates to the spatial arrangement of the undersampling scheme of the PAT. For example, the undersampling scheme may be defined by the particular K-space trajectory 620 used for sampling the K-space 600. Alternatively or additionally, the undersampling scheme may be defined with respect to the relative positioning of adjacent K-space position 610 for which MRI data is acquired, e.g., an offset along certain K-space directions. For example, a GRAPPA-scheme or CAIPIRINHA-scheme could be employed. In this regard, it would be possible that a given PAT scan parameter relates to the CAIPIRINHA offset of the undersampling scheme between adjacent K-space position 610 for which MRI data is acquired.

Based on the set of candidate values of the PATs scan parameters selected in block 5301, it is then possible to comprehensively model the undersampling scheme in block 5302. Additionally to PAT, it could also be possible that a MRI scan is considered which uses AC gradients applied along one or more phase-encoding directions during a readout event (cf. AC gradients 526 - 521, 536 - 538 according to the example of FIGs. 4, 5, and 12). In other words, it would be possible to employ Wave CAIPI techniques.

Accordingly, in block 5304, candidate values of Wave CAIPI scan parameters are selected. For example, candidate values could be selected for the amplitude, the phase shift, and/or the frequency / number of repetitions of the AC gradients.

Based on the imaging parameters and candidate properties, it is possible to model the point spread function (PSF) of the AC gradients with respect to the K-space position 610 during readout, i.e., the voxel spread.

Beyond such scan parameters related to PAT or Wave CAIPI, also further scan parameters conventionally associated with a MRI scan could be considered. Examples include a sampling bandwidth of the readout sequence; a resolution of the magnetic resonance imaging data; an oversampling factor of the readout sequence; the scan time; and the breath-hold time.

Based on the current candidate values for the various scan parameters, it is then possible to calculate the SNR characteristic of the MRI scan. For example, in block 5306 the so-called g-factor is calculated. The g-factor is indicative of the SNR of an image reconstructed from undersampled MRI data using a PAT. The g-factor may be indicative of artifacts resulting from the undersampled K-space. An example technique for calculating the g-factor in a spatially-resolved manner is known from Pruessmann, Klaas P., et al. "SENSE: sensitivity encoding for fast MRI." Magnetic resonance in medicine 42.5 (1999): 952-962 which is incorporated herein by reference in its entirety.

In some examples, the SNR characteristic may be determined spatially resolved, i.e., differently for different parts of the sample volume. In other examples, it would also be possible to determine the SNR characteristic in an integrated manner. This can be achieved, e.g., by taking the maximum or average of a spatially-resolved SNR characteristic.

Different candidate values will generally result in different SNR characteristics. Therefore, it can be desirable to recalculate the SNR characteristic for different sets of candidate values of the considered scan parameters. Accordingly, in block 5307 it is checked whether further candidate values of the scan parameters should be considered. For example, in block 5307 it would be possible to compare the SNR characteristic obtained with the current set of candidate values with a predefined threshold; if, for example, the SNR characteristic obtained with the current set of candidate values is below the threshold, then it can be judged in block 5307 that it is not necessary to calculate a further SNR characteristic using a further set of candidate values. Otherwise, one or more candidate values of the scan parameters may be adjusted in the next iteration of blocks 5301 - 5306.

In order to efficiently find a set of candidate values of the scan parameters which fulfills the various constraints and also provides an optimum SNR characteristic, it may be possible to perform an optimization. The optimization may help to appropriately adjust the candidate values from iteration to iteration of blocks 5301 - 5306 for fast convergence.

Although the invention has been shown and described with respect to certain preferred embodiments, equivalents and modifications will occur to others skilled in the art upon the reading and understanding of the specification. The present invention includes all such equivalents and modifications and is limited only by the scope of the appended claims.

For example, while various examples have been described for a 3-D MP RAGE MRI sequence, similar techniques may be readily employed for other types of 3-D MRI sequences, in particular other 3-D MRI sequences using an initial inversion pulse for magnetization preparation.

## Claims

1. A method, comprising:
- in a preparation phase: controlling a radio frequency transmitter of a magnetic resonance imaging device to apply an inversion pulse to a sample magnetization,
- in a multi-shot readout phase: controlling a gradient system of the magnetic resonance imaging device to apply a steady-state gradient echo readout sequence comprising at least one first phase-encoding gradient along a first direction, at least one second phase-encoding gradient along a second direction, and a sequence of readout gradients along a readout direction,
- in the multi-shot readout phase: controlling the gradient system to apply first AC gradients along the first direction and at least partly contemporaneously with readout gradients of the sequence of readout gradients,
- in the multi-shot readout phase: controlling the gradient system to apply second AC gradients along the second direction and at least partly contemporaneously with the readout gradients of the sequence of readout gradients, and
- in the multi-shot readout phase: controlling a radio frequency receiver of the magnetic resonance imaging device to acquire magnetic resonance imaging data for the sample magnetization at multiple K-space positions along a K-space trajectory.

2. The method of claim 1,
wherein subsequent K-space positions along the K-space trajectory are offset from each other in the first direction and in the second direction.

3. The method of claim 1,
wherein the readout sequence undersamples a plane defined by the first direction and the second direction in at least one of the first direction and the second direction.

4. The method of claim 1,
wherein nearest-neighbor K-space positions for which magnetic resonance imaging data are obtained are offset from each other in the first direction and in the second direction.

5. The method of claim 1,
wherein the K-space trajectory is zigzag-shaped in a plane defined by the first direction and the second direction.

6. The method of claim 1,
wherein the K-space trajectory comprises K-space positions having positive K values and negative K values along the first direction or along the second direction.

7. The method of claim 1,
wherein the magnetic resonance imaging data at a K-space position of the K-space trajectory corresponding to a K value of zero in the first direction or in the second direction is obtained adjacent to an inversion time of the sample magnetization defined by the inversion pulse.

8. The method of claim 1,
wherein the preparation phase and the readout phase are repeated multiple times using at least partly different phase-encoding gradients for different iterations,
wherein adjacent iterations are separated by a relaxation phase for the sample magnetization to recover.

9. A method, comprising:
- in a preparation phase: controlling a radio frequency transmitter of a magnetic resonance imaging device to apply an inversion pulse to a sample magnetization,
- in a multi-shot readout phase: controlling a gradient system of the magnetic resonance imaging device to apply a readout sequence comprising at least one first phase-encoding gradient along a first direction and at least one second phase-encoding gradient along a second direction, and
- in the multi-shot readout phase: controlling a radio frequency receiver of the magnetic resonance imaging device to acquire magnetic resonance imaging data for the sample magnetization at multiple K-space positions along a K-space trajectory,
wherein subsequent K-space positions along the K-space trajectory are offset from each other in the first direction and in the second direction.

10. The method of claim 9,
wherein the readout sequence is a steady-state gradient echo readout sequence comprising a sequence of readout gradients along a readout direction.

11. The method of claim 10, further comprising:
- in the multi-shot readout phase: controlling the gradient system to apply first AC gradients along the first direction and at least partly contemporaneously with readout gradients of the sequence of readout gradients, and
- in the multi-shot readout phase: controlling the gradient system to apply second AC gradients along the second direction and at least partly contemporaneously with the readout gradients of the sequence of readout gradients.

12. The method of claim 9,
wherein the readout sequence undersamples a plane defined by the first direction and the second direction in at least one of the first direction and the second direction.

13. The method of claim 9,
wherein nearest-neighbor K-space positions for which magnetic resonance imaging data is obtained are offset from each other in the first direction and in the second direction.

14. The method of claim 9,
wherein the K-space trajectory is zigzag-shaped in a plane defined by the first direction and the second direction.

15. The method of claim 9,
wherein the K-space trajectory comprises K-space positions having positive k values and negative k values along the first direction or along the second direction.

16. The method of claim 9,
wherein the magnetic resonance imaging data at a K-space position of the K-space trajectory corresponding to a K value of zero in the first direction or in the second direction is obtained adjacent to an inversion time of the sample magnetization defined by the inversion pulse.

17. The method of claim 9,
wherein the preparation phase and the readout phase are repeated multiple times using at least partly different phase-encoding gradients for different repetitions,
wherein adjacent repetitions are separated by a relaxation phase for the sample magnetization to recover.

18. A method, comprising:
- controlling a magnetic resonance imaging device to apply a 3-D MP RAGE magnetic resonance imaging sequence using Wave-CAIPI during a readout event.

19. A magnetic resonance imaging device, comprising:
- a radio frequency transmitter,
- a radio frequency receiver,
- a gradient system, and
- at least one processor configured to perform the following:
- in a preparation phase: controlling the radio frequency transmitter to apply an inversion pulse to a sample magnetization,
- in a multi-shot readout phase: controlling the gradient system to apply a steady-state gradient echo readout sequence comprising at least one first phase-encoding gradient along a first direction, at least one second phase-encoding gradient along a second direction, and a sequence of readout gradients along a readout direction,
- in the multi-shot readout phase: controlling the gradient system to apply first AC gradients along the first direction and at least partly contemporaneously with readout gradients of the sequence of readout gradients,
- in the multi-shot readout phase: controlling the gradient system to apply second AC gradients along the second direction and at least partly contemporaneously with the readout gradients of the sequence of readout gradients, and
- in the multi-shot readout phase: controlling the radio frequency receiver to acquire magnetic resonance imaging data for the sample magnetization at multiple K-space positions along a K-space trajectory.

20. A magnetic resonance imaging device, comprising:
- a radio frequency transmitter,
- a radio frequency receiver,
- a gradient system, and
- at least one processor configured to perform the following:
- in a preparation phase: controlling a radio frequency transmitter of a magnetic resonance imaging device to apply an inversion pulse to a sample magnetization,
- in a multi-shot readout phase: controlling a gradient system of the magnetic resonance imaging device to apply a readout sequence comprising at least one first phase-encoding gradient along a first direction and at least one second phase-encoding gradient along a second direction, and
- in the multi-shot readout phase: controlling a radio frequency receiver of the magnetic resonance imaging device to acquire magnetic resonance imaging data for the sample magnetization at multiple K-space positions along a K-space trajectory,
wherein subsequent K-space positions along the K-space trajectory are offset from each other in the first direction and in the second direction.

21. A method, comprising:
- retrieving a coil sensitivity map for at least some coils of a radio frequency coil assembly of a magnetic resonance imaging device,
- retrieving constraints for at least some of a plurality of scan parameters of a magnetic resonance imaging scan employing a parallel acquisition technique,
- based on the constraints and further based on the coil sensitivity map: predicting signal-to-noise characteristics of the magnetic resonance imaging scan for candidate values of the plurality of scan parameters,
- selecting values of the plurality of scan parameters from the candidate values based on the associated signal-to-noise characteristics, and
- based on the selected values: controlling the magnetic resonance imaging device to perform the magnetic resonance imaging scan.

22. The method of claim 21,
wherein the scan parameters of the plurality of scan parameters are selected from the group consisting of: an acceleration factor of the parallel acquisition technique; an undersampling scheme of the parallel acquisition technique; and a CAIPIRINHA offset of the undersampling scheme of the parallel acquisition technique.

23. The method of claim 21,
wherein the scan parameters of the plurality of scan parameters are selected from the group consisting of: an amplitude of AC gradients applied along a phase-encoding direction and at least partly contemporaneously with readout gradients; a phase shift between AC gradients applied along different phase-encoding directions and at least partly contemporaneously with readout gradients; and a frequency of AC gradients applied along at least one phase-encoding direction and at least partly contemporaneously with readout gradients.

24. The method of claim 21,
wherein the scan parameters of the plurality of scan parameters are selected from the group consisting of: a sampling bandwidth of a readout sequence; a resolution of magnetic resonance imaging data; an oversampling factor of a readout sequence; a scan time; and a breath-hold time.

25. The method of claim 21, further comprising:
- controlling the magnetic resonance imaging device to perform a reference magnetic resonance imaging scan using the radio frequency coil assembly to obtain reference magnetic resonance imaging data,
- controlling the magnetic resonance imaging device to perform a noise scan of the sample volume using the radio frequency coil assembly to obtain noise data,
- modifying the reference magnetic resonance imaging data based on the noise data, and
- determining the coil sensitivity maps based on the reference magnetic resonance imaging data.

26. The method of claim 21, further comprising:
- receiving the constraints for the at least some of the plurality of scan parameters from a human-machine interface.

27. A device comprising at least one processor configured to perform the following:
- retrieving a coil sensitivity map for at least some coils of a radio frequency coil assembly of a magnetic resonance imaging device,
- retrieving constraints for at least some of a plurality of scan parameters of an magnetic resonance imaging scan employing a parallel acquisition technique,
- based on the constraints and further based on the coil sensitivity map: predicting signal-to-noise characteristics of the magnetic resonance imaging scan for candidate values of the plurality of scan parameters,
- selecting values of the plurality of scan parameters from the candidate values based on the associated signal-to-noise characteristics, and
- based on the selected values: controlling the magnetic resonance imaging device to perform the magnetic resonance imaging scan.
